**Europäisches Patentamt**

**European Patent Office**

**Office européen des brevets**

(19)

(11) Publication number : **0 417 796 B1**

# EUROPEAN PATENT SPECIFICATION

(12)

(45) Date of publication of patent specification :
23.11.94 Bulletin 94/47

(51) Int. Cl.⁵ : **G01N 33/49**

(21) Application number : **90117678.4**

(22) Date of filing : **13.09.90**

(54) **Hematocrit measuring instrument.**

(30) Priority : **13.09.89 JP 237760/89**

(43) Date of publication of application :
**20.03.91 Bulletin 91/12**

(45) Publication of the grant of the patent :
**23.11.94 Bulletin 94/47**

(84) Designated Contracting States :
**DE FR GB SE**

(56) References cited :
**US-A- 4 484 135**
**US-A- 4 535 284**
**US-A- 4 692 685**
**PATENT ABSTRACTS OF JAPAN, vol. 12, no. 390 (P-772), 18th October 1988 ; & JP-A-63 133 062**

(73) Proprietor : **KABUSHIKI KAISHA TOYOTA CHUO KENKYUSHO**
**41-1, Aza Yokomichi**
**Oaza Nagakute**
**Nagakute-cho**
**Aichi-gun Aichi-ken, 480-11 (JP)**

(72) Inventor : **Ishihara, Toshikazu**
**Nagakute-ryo 203,41-3 Aza Yokomichi,**
**Oaza Nagakute**
**Nagakute-cho, Aichi-gun, Aichi-ken (JP)**

(74) Representative : **Blumbach Weser Bergen Kramer Zwirner Hoffmann Patentanwälte**
**Radeckestrasse 43**
**D-81245 München (DE)**

## Description

## BACKGROUND OF THE INVENTION

### 1. Field of the invention:

This invention relates to a hematocrit measuring instrument, more particularly to a hematocrit measuring instrument which is capable of performing highly accurate and continuous measurement even when there are variations in a blood temperature or in the concentration of the blood plasma electrolyte.

### 2. Description of the Related Art:

Measurement of the hematocrit of blood (the ratio of the volume of blood cells to the volume of the whole blood) is not only a very effective technique for the diagnosis of diseases, medical treatment and health care but also is a very effective technique to observe variations of the amount of the blood of patients who are undergoing blood purification treatment due to the use of artificial kidneys. Therefore there has been a strong demand for hematocrit measuring instruments which can perform highly accurate measurements continuously.

Various types of instruments have been known for hematocrit measurement including those employing centrifugation, those which make measurement from the blood cell count of a given volume (unit volume) of blood and the mean blood cell volume and those which make the measurement from the electrical resistance of the blood. Among the hematocrit measuring instruments, the type deriving the hematocrit based on the electrical resistance is characterized in that it can perform the measurement simply and continuously.

However, conventional hematocrit measuring instruments of the electrical resistance type suffer from measurement errors when an impedance of the blood plasma varies with the concentration of electrolyte or protein in the blood plasma.

The inventor of this invention has proposed a hematocrit measuring instrument in which impedances of the blood are measured by using two kinds of very different frequencies and the hematocrit is calculated based on the measured impedances (as disclosed in Japanese Patent JP-A-63 133 062.

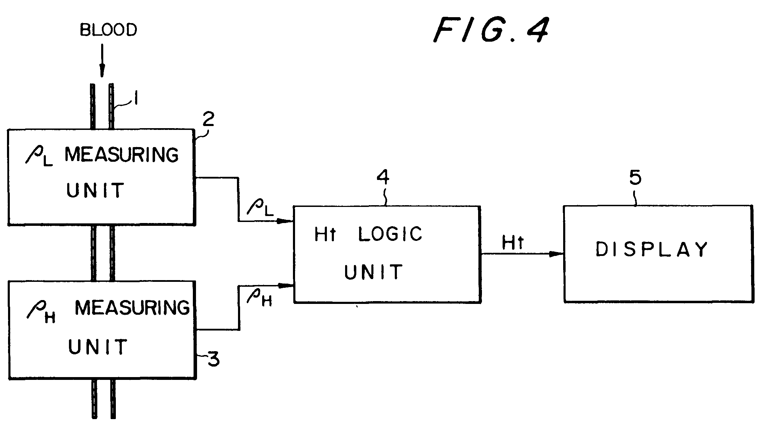

FIG. 4 of the accompanying drawings shows the conventional hematocrit measuring instrument mentioned above. This instrument is used to continuously measure the hematocrit of the blood in an extracorporeal circulation, for example in a path for hemodialysis. The conventional instrument comprises a low-frequency impedance measuring device 2 and a high-frequency impedance measuring device 3 which are disposed in a blood flow passage 1, and output measured values to an arithmetic instrument 4.

The low-frequency impedance measuring device 2 uses a low-frequency which makes an impedance of the cell membrane much larger than an impedance in blood plasma or intracellular fluid, and measures a low-frequency impedance $\rho_L$ of blood flowing through the blood flow passage 1.

The high-frequency impedance measuring device 3 uses a high-frequency which makes an impedance of cell membrane negligibly small compared with an impedance in blood plasma or intracellular fluid, and measures a high-frequency impedance $\rho_H$ of the blood flowing through the blood flow passage 1.

The arithmetic unit 4 calculates the hematocrit $H_t$ by applying the inputted values $\rho_L$ and $\rho_h$ to the relational expression between the low and high-frequency impedances of the blood and hematocrit, and displays the calculated value on a display 5.

The foregoing hematocrit measuring instrument is capable of carrying out very accurate measurements continuously even when there are variations in the concentration of the plasma electrolyte or protein.

With the conventional instrument, the impedance measuring devices 2, 3 are disposed at different positions in the blood flow passage 1, and the low and high-frequency impedances of the blood are measured at those positions. Therefore the following inconveniences have been experienced when it is desired to measure hematocrit more accurately.

The low and high-frequency impedances of the blood are strongly influenced not only by the concentration of plasma electrolyte or protein in the blood but also by a blood temperature. It is known from US-A-4 484 135 that the impedance of the blood varies with the temperature at a rate of about 2%/°C. If there is a temperature difference of one degree between the two impedance measuring points, about 1.5% error will be caused in the measured hematocrit value. It follows that accurate measurements cannot be carried out when there is a temperature difference between two measuring points.

Especially when an artificial kidney is controlled based on measured hematocrit during hemo-dialysis, for example, the measuring instrument is required to perform highly accurate measurement so as to obtain medical treatment with safty and high efficiency. The conventional measuring instrument has a problem that the hematocrit values are susceptible to errors caused by a change of blood temperature at the start of extracorporeal circulation, or ambient conditions under which medical treatment is being applied (e.g. due to the large difference between room temperature and body temperature).

In order to overcome the foregoing problems, there has been proposed an instrument in which temperature detectors such as thermistors are used to measure the blood temperatures at the two measuring points and variations in the measured blood impedances are compensated accordingly. However such a conventional instrument is very complicated as a whole, and when the blood temperature changes abruptly, suitable compensation cannot be carried out because there is a time lag in temperature detection. As a result, measured hematocrit values will become less accurate and less reliable.

## SUMMARY OF THE INVENTION

In the light of the problems of the conventional hematocrit measuring instrument, it is therefore an object of the invention to provide a simple hematocrit measuring instrument which is capable of continuously, quickly and accurately measuring hematocrit values based on the impedance of the blood without the need of measuring the blood temperature, for example.

The above object of the invention is accomplished by a hematocrit measuring instrument which comprises a blood flow passage, a low-frequency impedance measuring device in which at least a pair of low-frequency impedance measuring electrodes are disposed in the blood flow passage and are applied a low-frequency current in which a cell membrane impedance is much larger than a blood plasma impedance or an intra-cellular fluid impedance, so as to measure a low-frequency impedance of the blood, a high-frequency impedance measuring device in which at least a pair of high-frequency impedance measuring electrodes are disposed in the blood flow passage and are applied a high-frequency current in which the cell membrane impedance is negligible compared with the blood plasma impedance or the intracellular fluid impedance, so as to measure a high-frequency impedance in the blood, and an arithmetic unit for calculating a hematocrit value based on the low and high-frequency impedances of the blood. This hematocrit measuring instrument is characterized by a pair of low-frequency impedance measuring electrodes and a pair of high-frequency measuring electrodes which are disposed substantially at the same position in the blood flow passage in the blood flowing direction.

In the above configuration of the instrument, the low-frequency impedance measuring device is used for the following reason. Because a low-frequency current cannot pass through blood cells, the blood impedance representing a concentration of the blood cells, i.e., a hematocrit value, can be measured.

A high-frequency current is used for the following reasons. Since a high-frequency current can freely pass through the blood cells, the blood impedance representing the concentration of the electrolyte or protein in the blood can be measured independently of the hematocrit value. Therefore, the low-frequency impedance of the blood can be adjusted to derive the hematocrit value accurately based on the measured blood impedance value.

In addition, the reason why both low and high-frequency impedances are measured substantially at the same point of the blood flow passage is that variations of impedances in the blood due to blood temperature change are compensated by measuring the impedances at the same temperature. In short, the blood impedances vary greatly with temperatures as described previously. The temperature characteristics of the blood impedance depend upon the temperature characteristics of electric conduction of ions such as Na+, Cl- which are carriers of electric conduction in the blood. Therefore, the impedances of the blood vary nearly at the same ratio regardless of a low or high-frequency when a blood temperature varies. To sum up, the impedance of the blood decreases by about 2%/°C for the low and high frequencies.

Upon analyzing the frequency characteristics of the blood impedance, it is known that the hematocrit is a function of the ratio between the low-frequency impedance $\rho_L$ and high-frequency impedance $\rho_H$, i.e., $\rho_L/\rho_H$.

The low-frequency impedance $\rho_L$ and high-frequency impedance $\rho_H$ of the blood vary with the blood temperature. However if these impedances are measured at the same temperature, the ratio $\rho_H/\rho_L$ is not influenced by the temperature. The hematocrit which is the function of the ratio $\rho_L/\rho_H$ can be calculated without influence of the temperature.

The operation of the hematocrit measuring instrument will be described hereinafter.

With the instrument of the invention, impedances of the blood are measured substantially at the same point in the blood flow passage by using two kinds of different frequencies, so that the hematocrit can be calculated based on the measured impedances.

The invention is conceived based on the temperature characteristics and the frequency characteristics of the impedance of the blood. A low-frequency impedance measuring device and a high-frequency impedance measuring device are disposed substantially at the same position on the blood flow passage, and a low-frequency impedance and a high-frequency impedance of the blood are measured at the same temperature. The measured values are inputted to an arithmetic unit.

Based on the inputted impedances, the arithmetic unit calculates the hematocrit of the blood after compensating for the temperature dependability of the impedances, and outputs the hematocrit value.

According to the invention, two kinds of impedances, i.e., a low and a high-frequency impedance of blood are measured at the same temperature so as to calculate the hematocrit.

The hematocrit measuring instrument of the invention can continuously measure the hematocrit without any influence of the blood temperature, or concentration of plasma electrolyte or protein. In addition, the measuring instrument is capable of accurate measurement of the hematocrit even when medical fluid containing electrolyte is applied in the blood, or even when the blood temperature or concentration of plasma electrolyte changes abruptly during a blood purification process, for example.

Since it is not necessary to measure the temperature, the measuring instrument does not need thermometers or temperature compensators, which will make the instrument simple and inexpensive.

It is preferable that the low-frequency impedance measuring device of the invention measures a low-frequency impedance of the blood by applying a low-frequency current of 5 $KH_Z$ to 50 $KH_Z$, while the high-frequency impedance measuring device measures a high-frequency impedance of the blood by using a high-frequency current of 20 $MH_Z$ to 200 $MH_Z$.

The aforementioned low-frequency of 5$KH_Z$ to 50$KH_Z$ is selected because it is safe for the human body, the current flowing through the blood cell is very weak, and no electrode polarization is caused, so that the accurate hematocrit values can be obtained.

The high-frequency of 20 $MH_Z$ to 200 $MH_Z$ is selected because this frequency can pass through the cell membranes of the blood very easily, and variations of the concentration of the plasma electrolyte or protein can be precisely measured independently of the hematocrit.

In the hematocrit measuring instrument of the invention, it is preferable that the arithmetic unit derives the hematocrit value $H_t$ from the following formula.

$$H_t = K \log_e(\rho_L/\rho_h)$$

where $\rho_L$ represents a low-frequency impedance; $\rho_h$: a high-frequency impedance; and k: a constant.

The hematocrit value can be accurately derived from the foregoing formula because the formula for measuring a hematocrit, a low-frequency impedance and a high-frequency impedance of a patient's blood is identical to the aforementioned formula.

In the hematocrit measuring instrument of the invention, the electrodes for measuring both low and high frequencies are disposed in the blood flow passage so as to allow the currents to intersect with each other so that very minute mutual interference of both low and high-frequency current can be minimized. In addition, the very minute mutual interference is removed by a low-pass filter and a high-pass filter, so that both the low and high-frequency impedances can be accurately measured at the same temperature.

One of the two high-frequency electrodes is used also as a low-frequency electrode so as to decrease the total number of the electrodes and to simplify the structure of a resistivity measurement cell. In addition, it is possible to measure the impedances of the blood at the same temperature when the measurement is performed at the positions which are very close to each other.

## BRIEF DESCRIPTION OF THE DRAWINGS

FIG. 1 is a block diagram of a hematocrit measuring instrument according to a first embodiment of the invention;

FIG. 2 is a diagram showing the structure of a low-frequency measuring device and a high-frequency measuring device employed in a second embodiment of the invention;

FIG. 3 is a diagram showing a hematocrit measuring instrument according to a third embodiment of the invention; and

FIG. 4 is a diagram showing a conventional hematocrit measuring device.

## DETAILED DESCRIPTION

Embodiment 1:

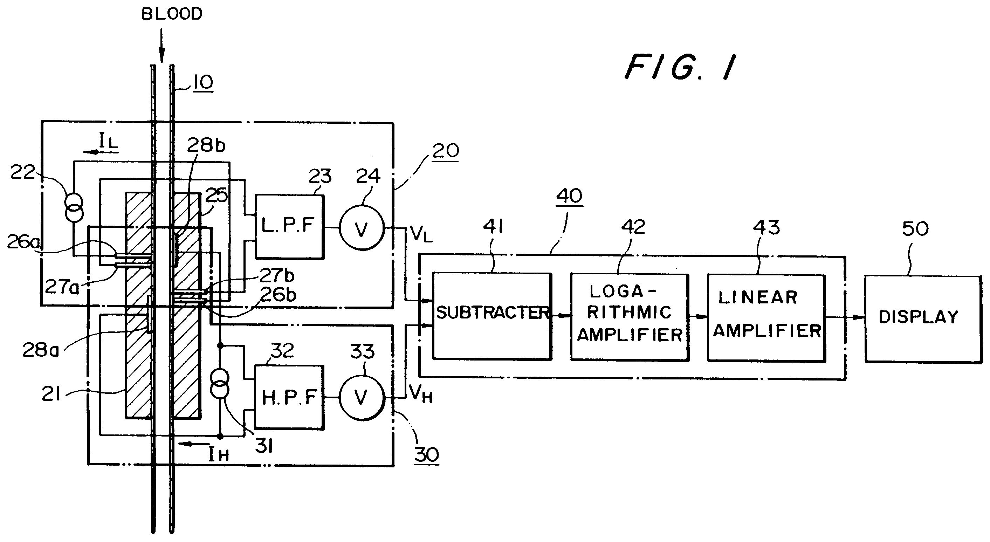

FIG. 1 shows a basic structure of the hematocrit measuring instrument, which continuously measures the hematocrit of blood in a circulatory path outside the body, for example in a path for hemo-dialysis. Both a low-frequency impedance measuring device 20 and a high-frequency impedance measuring device 30 are disposed as a unit in a blood flow passage 10.

The first embodiment of the hematocrit measuring instrument is characterized in that both a low-frequency impedance $\rho_L$ and a high-frequency impedance $\rho_H$ of the blood are measured by the measuring devices 20 and 30 substantially at the same position in the blood flow passage 10. Then the hematocrit is continuously measured based on the measured values $\rho_L$ and $\rho_H$.

In this embodiment, the low-frequency impedance measuring device 20 includes a resistivity measurement cell 21, a low-frequency constant current source 22, a low-pass filter 23 and an AC voltmeter 24.

The high-frequency impedance measuring device 30 includes the resistivity measurement cell 21 to be also used by the low-frequency impedance measuring device 20, a high-frequency constant current source 31, a high-pass filter 32 and an AC voltmeter 33.

The resistivity measurement cell 21 comprises an insulative cylindrical polycarbonate pipe 25, at the center of which a blood flow passage 10 is disposed. The pipe 25 has an electrode 26a and an electrode 26b. The electrode 26a is disposed facing the blood flow passage 10 while the other electrode 26b is disposed across the blood flow passage 10 at a position which is on the opposite side of the electrode 26a and is at a certain distance from the electrode 26a in the direction of the central axis of the blood flow passage 10. The pair of electrodes 26a, 26b serve as the low-frequency current source for measuring the low-frequency impedance.

A pair of voltage pickup electrodes 27a, 27b are disposed along the blood flow passage 10 inside the electrodes 26a, 26b, respectively.

In addition, the pipe 25 has electrodes 28a and 28b which face the blood flow passage 10. The electrode 28a is on the side where the electrode 26a is disposed. An electrode 28b is on the side where the electrode 26b is disposed. The pair of the electrodes 28a, 28b are electrodes for measuring the high-frequency impedance. The electrodes 28a, 28b are disposed so that the high-frequency current path and the low-frequency current path intersect with each other in the pipe 25 by means of the electrodes 26a and 26b.

The pair of the low-frequency electrodes 26a, 26b are connected to the low-frequency constant current source 22, which supplies these electrodes 26a, 26b with a low-frequency AC current $I_L$ of 50 KH$_z$, 100 μA r.m.s. which cannot pass through blood cells, does not cause electrode polarization, and does not give any harm to the human body.

The pair of the high-frequency electrodes 28a, 28b are connected to the high-frequency current source 31, which supplies the electrodes 28a, 28b with a high current $I_H$ of 20 MH$_z$, 100 μA r.m.s. which can easily pass through the blood cells and does not give any harm to the human body. The low-frequency current $I_L$ and the high-frequency current $I_H$ are supplied in the direction so that they intersect with each other in the blood flow passage by means of the electrodes 28a, 28b.

Although the two kinds of the measurement currents flow substantially at the same position in the blood flow passage 10, a 50 KH$_z$ AC voltage proportional to the impedance $\rho_L$ of the blood can be generated between the voltage pickup electrodes 27a, 27b (adjacent to the low-frequency current supply electrodes 26a, 26b) while suppressing generation of a 20 MH$_z$ AC voltage as little as possible.

On the contrary, the 20 MH$_z$ AC voltage proportional to the impedance $\rho_H$ of the blood can be generated between the high-frequency electrodes 28a, 28b while suppressing generation of the 50 KH$_z$ AC voltage.

The low-pass filter 23 prevents flow of the 20 MH$_z$ AC signal from the voltage pickup electrodes 27a, 27b, and outputs the 50 KH$_z$ AC signal to the AC volt-meter 24.

The high-pass filter 32 prevents flow of the 50 KH$_z$ AC signal from the high-frequency electrodes 28a, 28b, and outputs the 20 MH$_z$ AC voltage to the AC volt-meter 33.

The AC voltmeters 24 and 33 respectively-convert the AC voltages from the low- and high-pass filters 23, 32 into DC voltages $V_L$ and $V_H$, and output them to the hematocrit arithmetic unit 40.

According to this embodiment, since the two kinds of the measurement currents are supplied in the directions which intersect with each other, both the low and high-frequency impedances of the blood can be measured at the same point in the blood flow passage without any adverse influence with each other. In addition, residual noise components can be eliminated by the low- and high-pass filters, so that the low-frequency and high-frequency impedances of the blood can be measured accurately without any interference between them.

In addition, the low-frequency current $I_L$ cannot pass easily through the cell membranes of the blood cells. Although anisotrophy may occur in the low-frequency impedance of the blood due to the orientation of the blood cells, this can be prevented by the following measures. The low-frequency impedance measurement device 20 has four electrodes and measures the impedance in the oblique direction of the blood flow passage as a low-frequency impedance of the blood. Therefore the impedance of the blood can be accurately measured without any influence of the anisotropy of the blood impedance caused by the orientation of the blood cells.

The hematocrit arithmetic unit 40 calculates the hematocrit of the blood based on the impedances $\rho_L$ and $\rho_H$ of the blood measured by the measurement devices 20, 30, and comprises a divider 41, a logarithmic amplifier 42 and an ordinary linear amplifier 43.

The divider 41 divides the voltage $V_L$, pro portional to the impedance $\rho_L$ at 50 KH$_z$ of the blood, with the voltage $V_H$ proportional to the impedance $\rho_H$ at 20 MH$_z$, and outputs the ratio $\gamma = V_L/V_H = (\rho_L/\rho_H)$ as a voltage signal to the logarithmic amplifier 42.

The logarithmic amplifier 42 converts the signal $\gamma$ from the divider 41 into a logarithm $\log_e\gamma$. The linear amplifier 43 multiplies $\log_e\gamma$ by K times, and outputs the derived value to a display 50 as the hematocrit value $H_t = K \log_e\gamma$.

It is preferable that the amplification factor K be 88 in the linear amplifier 43.

The display 50 includes an A/D converter and a display panel, converts the analog voltage signal from the hematocrit arithmetic unit 40 into digital signals and gives a digital indication of the hematocrit value $H_t = 88 \log_e (\rho_L/\rho_H)$ in the unit of %.

As described above, the low and high-frequency impedances $\rho_L$ and $\rho_H$ of the blood are measured substantially at the same point of the blood flow passage 10 at the same temperature, thereby enabling the hematocrit value to be accurately measured without being influenced by temperature variations. Therefore, the hematocrit value can be accurately measured even when the blood flow speed or the room temperature changes, and the temperature of the blood or concentration of the electrolyte in the blood plasma changes abruptly since medical fluid containing an electrolyte is applied to the patient's blood, for example.

In addition, both the low and high-frequency impedances $\rho_L$ and $\rho_H$ are measured at the same temperature so as to nullify any influence of the temperature variation. There will be no need for a thermistor or a device for correcting the measured values according to the temperature variation. This enables the whole measuring instrument to be simple and inexpensive.

Embodiment 2:

The resistivity measurement cell which is directly in contact with the patient's blood should be preferably disposable so as to prevent infection of viruses, germs or the like. Such disposable members should be simple and inexpensive. A second embodiment of the invention is contemplated for such a purpose. FIG. 2 shows a hematocrit measuring instrument for meeting the above-described request.

In this embodiment, the hematocrit arithmetic unit and the display are the same as those employed in the first embodiment, and will not be described hereinafter. The members similar to those of the embodiment 1 will be assigned the same reference numerals.

The measuring instrument in this embodiment com prises the resistivity measurement cell 21 and a combined unit of the low and high-frequency impedance measuring devices 20 and 30 in the same manner as in the first embodiment.

The measuring instrument is characterized in that one of two low-frequency current supply electrodes 26a, 26b (26b in this embodiment) is also used as the high-frequency electrode 28b so as to decrease the number of the electrodes used, thereby simplifying the resistance measurement cell 21.

The low-frequency current supply electrodes 26a, 26b and the voltage pickup electrodes 27a, 27b are disposed on the insulative pipe 25 as in the Embodiment 1. The high-frequency supply electrode 28b also serves as the low-frequency current supply electrode 26b same as in the Embodiment 1.

The electrode 26b and 28a are connected to the high-frequency constant current source 31 and the high-pass filter 32 so as to measure the high-frequency impedance of the blood.

The hematocrit measuring instrument of the above configuration can flow both the low and high-frequency currents in the direction which intersect with each other at the perpendicular direction in the vicinity of the blood flow passage 10, and can measure the low and high-frequency impedances $\rho_L$ and $\rho_H$ of the blood.

In the above embodiment, the electrode 26b is used for measuring both of the low and high-frequency impedances of the blood by way of the first embodiment. The electrode 26a is also usable for this purpose.

Embodiment 3:

In the foregoing embodiments, the pair of the low-frequency current supply electrodes 26a, 26b are disposed at positions which are separated in the direction of the central axis of the insulative pipe 25, and the high-frequency electrodes 28a, 28b are also disposed at positions which are separated in the direction along the central axis of the pipe 25. Therefore the low-frequency current path formed by the electrodes 26a, 26b intersect with the high-frequency current path formed by the electrodes 28a, 28b.

In this embodiment, the electrodes are not necessarily disposed as described above, and are disposed substantially at the same point in the direction of the blood flow.

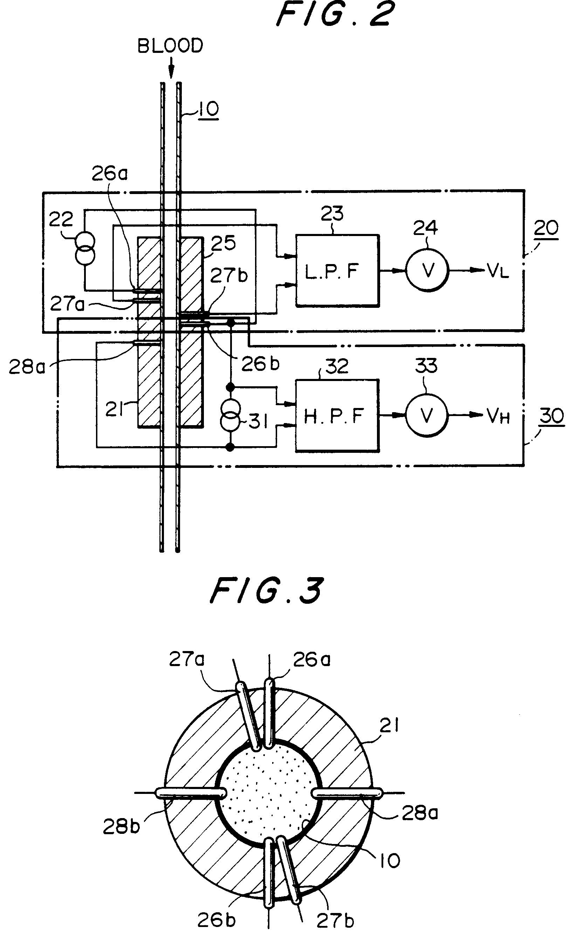

FIG. 3 shows an embodiment of the arrangement of the electrodes, and is a sectional view of the resistivity measurement cell 21, cut in the plane where the cell 21 is perpendicular to the blood flow passage 10.

The low-frequency current supply electrodes 26a, 26b are disposed on the opposite sides of the blood flow passage 10, and the voltage pickup electrodes 27a, 27b are disposed in the vicinity of the electrodes 26a, 26b.

The high-frequency electrodes 28a, 28b are disposed on the opposite sides of the blood flow passage 10 so that the high-frequency current path via the electrodes 28a, 28b intersect the low-frequency current path via the electrodes 26a, 26b.

As described with reference to the Embodiments 1 and 2, both of the low and high-frequency impedances of the blood can be measured substantially at the same point of the blood flow passage 10 at the same temperature, so that the hematocrit value can be accurately measured without any influence of the temperature variation of the blood.

The explanation of the other members will be omitted since they are similar as those in the embodiment 1.

With the foregoing embodiments, the low- and high-pass filters are used so as to prevent the mutual interference between the low and high-frequency impedances. This invention is not limited to the use of these filters, but the interference between the low and high-frequency impedances can be eliminated by measuring the impedances on the time-sharing basis, for example.

The above embodiments are described about the hematocrit measuring instrument to be used for measurement by using a circulatory path outside the human body. However the instrument is not limited to the use mentioned above, but also is applicable to the hematocrit measurement of collected blood by modifying shapes of the low and high-frequency impedance measuring devices, for example.

## Claims

1. A hematocrit measuring instrument for deriving a hematocrit value from the impedance of the blood and comprising:

    (a) a blood flow passage (10);

    (b) a low-frequency impedance measuring device (20) in which at least a pair of low-frequency impedance measuring electrodes (26a/26b, 27a/27b) are disposed in said blood flow passage and are applied a low-frequency current in which a cell membrane impedance is much larger than a blood plasma impedance or an intra-cellular fluid impedance, so as to measure a low-frequency impedance of the blood;

    (c) a high-frequency impedance measuring device (30) in which at least a pair of high-frequency impedance measuring electrodes (28a/28b) are disposed in said blood flow passage and are applied a high-frequency current in which the cell membrane impedance is negligible compared with the blood plasma impedance or the intracellular fluid impedance, so as to measure a high-frequency impedance in the blood; and

    (d) an arithmetic unit (40) for calculating a hematocrit value based on the low and high-frequency impedances of the blood; characterized in that said pair of low-frequency impedances measuring electrodes (26a/26b, 27a/27b) and said pair of high-frequency impedance measuring electrodes (28a/28b) are disposed substantially at the same position in said blood flow passage in the blood flowing direction.

2. A hematocrit measuring instrument according to claim 1, wherein one of said low-frequency impedance measuring electrodes (26a, 27a) is disposed facing said blood flow passage (10) while the other low-frequency impedance measuring electrode (26b, 27b) is disposed across said blood flow passage (10) at a position which is on the opposite side of said low-frequency impedance measuring electrode and is at a certain distance from said electrode (26a, 27a) in the direction of the central axis of said blood flow passage; and one of said high-frequency impedance measuring electrodes (28a) is disposed facing said blood flow passage (10) while the other high-frequency impedance measuring electrode (28b) is disposed across said blood flow passage (10) at a position which is on the opposite side of said high-frequency impedance measuring electrode and is at a certain distance from said electrode in the direction of the central axis of said blood flow passage, so that said high-frequency current flow passage through said high-frequency impedance measuring electrodes and said low-frequency current flow passage through said low-frequency measuring electrodes intersect with each other in said blood flow passage.

3. A hematocrit measuring instrument according to claim 1, wherein both of said low and high-frequency impedance measuring electrode pairs are disposed so that their low-frequency current passage and high-frequency current passage intersect in a plane which is perpendicular to the central axis of said blood flow passage.

4. A hematocrit measuring instrument according to any of claims 1 to 3, wherein both of said low and high-frequency impedance measuring electrode pairs are disposed on an insulated resistance measuring cell (21) in said blood flow passage (10).

5. A hematocrit measuring instrument according to any of claims 1 to 4, wherein said low-frequency impedance measuring device includes a low-frequency constant current source (22) for supplying a low-frequency current to the blood through said pair of low-frequency impedance measuring electrodes (26a/26b, 27a/27b), a low pass filter (23) for passing a low-frequency voltage signal generated between said pair of low-frequency impedance measuring electrodes (26a/26b, 27a/27b), and low-frequency impedance outputting means (24) for measuring a voltage of the low-frequency voltage signal outputted from said low-pass filter and for outputting a signal indicating the low-frequency impedance of the blood; and said high-frequency impedance measuring device includes a high-frequency constant current source (31) for supplying a high-frequency current to the blood through said pair of high-frequency impedance measuring electrodes (28a/28b), a high-pass filter (32) for passing a high-frequency voltage signal generated between said pair of high-frequency impedance measuring electrodes (28a/28b), and high-frequency impedance outputting means (33) for measuring a voltage of the high-frequency voltage signal outputted from said high-pass filter and for outputting a signal indicating the high-frequency impedance of the blood.

6. A hematrocrit measuring instrument according to claim 5, wherein said low-frequency impedance measuring device (20) includes a pair of voltage detecting electrodes (27a/27b) disposed adjacent to said pair of low-frequency impedance measuring electrodes (26a/26b) and connected to said low-pass filter.

7. A hematocrit measuring instrument according to any of claims 1 to 6, wherein at least one of said pair of high-frequency impedance measuring electrodes is also used as a low-frequency impedance measuring electrode so as to decrease the number of said electrodes.

8. A hematocrit measuring instrument according to any of claims 1 to 7, wherein said low-frequency impedance measuring device measures a low-frequency impedance of the blood by means of a low-frequency of 5 $KH_Z$ to 50 $KH_Z$, and said high-frequency impedance measuring device measures a high-frequency impedance of the blood by means of a high-frequency of 20 $MH_Z$ to 200 $MH_Z$.

**Patentansprüche**

1. Hämatokrit-Meßgerät zum Herleiten eines Hämatokritwertes aus der Impedanz des Blutes, welches aufweist:

   (a) einen Blutkanal (10);
   (b) eine Niederfrequenzimpedanzmeßvorrichtung (20), bei der mindestens ein Paar von Niederfrequenzimpedanzmeßelektroden (26a/26b, 27a/27b) in dem Blutkanal (10) angeordnet und mit einem Niederfrequenzstrom beaufschlagt ist, bei dem die Impedanz der Zellmembran wesentlich größer als die Impedanz des Blutplasmas oder die Impedanz der Zellflüssigkeit ist, um die Niederfrequenzimpedanz des Blutes zu messen;
   (c) eine Hochfrequenzimpedanzmeßvorrichtung (30), bei der mindestens ein Paar von Hochfrequenzimpedanzmeßelektroden (28a/28b) in dem Blutkanal angeordnet und mit einem Hochfrequenzstrom beaufschlagt ist, bei dem die Impedanz der Zellmembran im Vergleich zur Impedanz des Blutplasmas oder der Impedanz der Zellflüssigkeit vernachlässigbar ist, um die Hochfrequenzimpedanz im Blut zu messen; und
   (d) eine Arithmetikeinheit (40) zum Berechnen des Hämatokritwertes auf der Grundlage der Nieder- und Hochfrequenzimpedanzen des Blutes;
   dadurch **gekennzeichnet**, daß das Paar von Niederfrequenzimpedanzmeßelektroden (26a/26b, 27a/27b) und das Paar von Hochfrequenzmeßelektroden (28a/28b) im wesentlichen an derselben Stelle des Blutkanales (10) in Blutflußrichtung angeordnet sind.

2. Hämatokrit-Meßgerät nach Anspruch 1, bei dem eine der Niederfrequenzimpedanzmeßelektroden (26a, 27a) gegenüber dem Blutkanal (10) angeordnet ist, und die andere Niederfrequenzimpedanzmeßelektrode (26b, 27b) quer zu dem Blutkanal (10) an einer Stelle angeordnet ist, die sich auf der gegenüberliegenden Seite der Niederfrequenzimpedanzmeßelektrode und in einem bestimmten Abstand von dieser Elektrode (26a, 27a) in der Richtung der Mittelachse des Blutkanales befindet; und eine der Hochfrequenzimpedanzmeßelektroden (28a) gegenüber dem Blutkanal (10) angeordnet ist, und die andere Hochfrequenzimpedanzmeßelektrode (28b) quer zu dem Blutkanal (10) an einer Stelle angeordnet ist, die sich auf der gegenüberliegenden Seite der Hochfrequenzimpedanzmeßelektrode und in einem bestimmten Abstand von dieser Elektrode in der Richtung der Mittelachse des Blutkanales befindet, so daß sich der Hochfrequenzstromkanal durch die Hochfrequenzimpedanzmeßelektroden und der Niederfrequenzstromkanal durch die Niederfrequenzimpedanzmeßelektroden in dem Blutkanal schneiden.

3. Hämatokrit-Meßgerät nach Anspruch 1, bei dem beide Nieder- und Hochfrequenzimpedanzmeßelektrodenpaare derart angeordnet sind, daß sich deren Niederfrequenzstrompfad und Hochfrequenzstrompfad in einer Ebene schneiden, die senkrecht zur Mittelachse des Blutkanales liegt.

4. Hämatokrit-Meßgerät nach einem der Ansprüche 1 bis 3, bei dem beide Nieder- und Hochfrequenzimpedanzmeßelektrodenpaare auf einer isolierenden Widerstandsmeßzelle (21) in dem Blutkanal (10) angeordnet sind.

5. Hämatokrit-Meßgerät nach einem der Ansprüche 1 bis 4, bei dem die Niederfrequenzimpedanzmeßvorrichtung eine Niederfrequenz-Konstantstromquelle (22) zum Liefern eines Niederfrequenzstromes an das Blut durch das Paar der Niederfrequenzimpedanzmeßelektroden (26a/26b, 27a/27b), ein Tiefpaßfilter (23) zum Durchlassen des zwischen dem Paar von Niederfrequenzimpedanzmeßelektroden (26a/26b, 27a/27b) erzeugten Niederfrequenz-Spannungssignals und eine Niederfrequenz-Ausgabeeinrichtung (24) zum Messen der Spannung des von dem Tiefpaßfilter ausgegebenen Niederfrequenz-Spannungssignales und zum Ausgeben eines Signales, das die Niederfrequenzimpedanz des Blutes anzeigt, aufweist; und die Hochfrequenzimpedanzmeßvorrichtung eine Hochfrequenz-Konstantstromquelle (31) zum Liefern eines Hochfrequenzstromes an das Blut durch das Paar von Hochfrequenzimpedanzmeßelektroden (28a/28b), ein Hochpaßfilter (32) zum Durchlassen des zwischen dem Paar von Hochfrequenzimpe-

danzmeßelekt roden (28a/28b) erzeugten Hochfrequenz-Spannungssignales und eine Hochfrequenzimpedanz-Ausgabeeinrichtung (33) zum Messen der Spannung des von dem Hochpaßfilter ausgegebenen Hochfrequenz-Spannungssignales und zum Ausgeben eines Signales, das die Hochfrequenz-8impedanz des Blutes anzeigt, aufweist.

6. Hämatokrit-Meßgerät nach Anspruch 5, bei dem die Niederfrequenzimpedanzmeßvorrichtung (20) ein Paar von Spannungserfassungselektroden (27a, 27b), die angrenzend zu dem Paar von Niederfrequenzimpedanzmeßelektroden (26a/26b) angeordnet und mit dem Tiefpaßfilter verbunden sind, aufweist.

7. Hämatokrit-Meßgerät nach einem der Ansprüche 1 bis 6, bei dem mindestens eine des Paares von Hochfrequenzimpedanzmeßelektroden auch als Niederfrequenzimpedanzmeßelektrode verwendet ist, um die Anzahl der Elektroden zu senken.

8. Hämatokrit-Meßgerät nach einem der Ansprüche 1 bis 7, bei dem die Niederfrequenzimpedanzmeßvorrichtung mittels einer Niederfrequenz von 5 kHz bis 50 kHz die Niederfrequenzimpedanz des Blutes mißt, und die Hochfrequenzimpedanzmeßvorrichtung mittels einer Hochfrequenz von 20 MHz bis 200 MHz die Hochfrequenzimpedanz des Blutes mißt.

**Revendications**

1. Un instrument de mesure d'hématocrite destiné à déterminer une valeur d'hématocrite à partir de l'impédance du sang et comprenant :
   (a) un passage d'écoulement de sang (10);
   (b) un dispositif de mesure d'impédance en basse fréquence (20) dans lequel au moins une paire d'électrodes de mesure d'impédance en basse fréquence (26a/26b, 27a/27b) sont disposées dans le passage d'écoulement de sang et reçoivent un courant de basse fréquence pour lequel l'impédance de la membrane d'une cellule est très supérieure à l'impédance du plasma sanguin ou à l'impédance du fluide intra-cellulaire, de façon à mesurer une impédance du sang en basse fréquence;
   (c) un dispositif de mesure d'impédance en haute fréquence (30), dans lequel au moins une paire d'électrodes de mesure d'impédance en haute fréquence (28a/28b) sont disposées dans le passage d'écoulement de sang et reçoivent un courant de haute fréquence, pour lequel l'impédance de la membrane d'une cellule est négligeable vis-à-vis de l'impédance du plasma sanguin ou de l'impédance du fluide intra-cellulaire, de façon à mesurer une impédance du sang en haute fréquence; et
   (d) une unité arithmétique (40) pour calculer une valeur d'hématocrite sur la base des impédances du sang en basse fréquence et en haute fréquence;
   caractérisé en ce que la paire d'électrodes de mesure d'impédance en basse fréquence (26a/26b, 27a/27b) et la paire d'électrodes de mesure d'impédance en haute fréquence (26a/28b) sont disposées pratiquement à la même position dans le passage d'écoulement de sang, dans la direction d'écoulement du sang.

2. Un instrument de mesure d'hématocrite selon la revendication 1, dans lequel une première des électrodes de mesure d'impédance en basse fréquence (26a, 27a) est disposée face au passage d'écoulement de sang (10), tandis que l'autre électrode de mesure d'impédance en basse fréquence (26b, 27b) est disposée de l'autre côté du passage d'écoulement de sang (10) à une position qui est du côté opposé à celui de la première électrode de mesure d'impédance en basse fréquence et qui est à une certaine distance de cette électrode (26a, 27a) dans la direction de l'axe central du passage du passage d'écoulement de sang; et une première des électrodes de mesure d'impédance en haute fréquence (28a) est disposée face au passage d'écoulement de sang (10), tandis que l'autre électrode de mesure d'impédance en haute fréquence (28b) est disposée de l'autre côté du passage d'écoulement de sang (10), à une position qui est du côté opposé à celui de l'électrode de mesure d'impédance en haute fréquence et qui est à une certaine distance de cette électrode dans la direction de l'axe central du passage d'écoulement de sang, de façon que le chemin de circulation du courant de haute fréquence entre les électrodes de mesure d'impédance en haute fréquence et le chemin de circulation du courant de basse fréquence entre les électrodes de mesure d'impédance en basse fréquence se coupent mutuellement dans le passage d'écoulement de sang.

3. Un instrument de mesure d'hématocrite selon la revendication 1, dans lequel les paires d'électrodes de mesure d'impédance en basse fréquence et en haute fréquence sont disposées de façon que leur chemin de circulation du courant de basse fréquence et leur chemin de circulation du courant de haute fréquence se coupent dans un plan qui est perpendiculaire à l'axe central du passage d'écoulement de sang.

4. Un instrument de mesure d'hématocrite selon l'une quelconque des revendications 1 à 3, dans lequel les paires d'électrodes de mesure d'impédance en basse fréquence et en haute fréquence sont disposées sur une cellule de mesure de résistance isolée (21) dans le passage d'écoulement de sang (10).

5. Un instrument de mesure d'hématocrite selon l'une quelconque des revendications 1 à 4, dans lequel le dispositif de mesure d'impédance en basse fréquence comprend une source de courant constant de basse fréquence (22) qui est destinée à faire circuler un courant de basse fréquence dans le sang entre les électrodes de la paire d'électrodes de mesure d'impédance en basse fréquence (26a/26b, 27a/27b), un filtre passe-bas (23) pour transmettre un signal de tension de basse fréquence qui est généré entre les électrodes de la paire d'électrodes de mesure d'impédance en basse fréquence (26a/26b, 27a/27b), et des moyens d'émission de valeur d'impédance en basse fréquence (24) pour mesurer la tension du signal de tension de basse fréquence qui est émis par le filtre passe-bas, et à émettre un signal indiquant l'impédance du sang en basse fréquence; et le dispositif de mesure d'impédance en haute fréquence comprend une source de courant constant de haute fréquence (31) qui est destinée à faire circuler un courant de haute fréquence dans le sang, entre les électrodes de la paire d'électrodes de mesure d'impédance en haute fréquence (26a/28b), un filtre passe-haut (32) qui est destiné à transmettre un signal de tension de haute fréquence qui est généré entre les électrodes de la paire d'électrodes de mesure d'impédance en haute fréquence (28a/28b), et des moyens d'émission de valeur d'impédance en haute fréquence (33) destinés à mesurer la tension du signal de tension de haute fréquence qui est émis par le filtre passe-haut, et à émettre un signal indiquant l'impédance du sang en haute fréquence.

6. Un instrument de mesure d'hématrocrite selon la revendication 5, dans lequel le dispositif de mesure d'impédance en basse fréquence (20) comprend une paire d'électrodes de détection de tension (27a/27b) qui sont disposées en position adjacente à la paire d'électrodes de mesure d'impédance en basse fréquence (26a/26b) et qui sont connectées au filtre passe-bas.

7. Un instrument de mesure d'hématocrite selon l'une quelconque des revendications 1 à 6, dans lequel l'une au moins des électrodes de la paire d'électrodes de mesure d'impédance en haute fréquence est également utilisée à titre d'électrode de mesure d'impédance en basse fréquence, de façon à diminuer le nombre des électrodes.

8. Un instrument de mesure d'hématocrite selon l'une quelconque des revendications 1 à 7, dans lequel le dispositif de mesure d'impédance en basse fréquence mesure l'impédance du sang en basse fréquence au moyen d'une basse fréquence de 5 kHz à 50 kHz, et le dispositif de mesure d'impédance en haute fréquence mesure l'impédance du sang en haute fréquence au moyen d'une haute fréquence de 20 MHz à 200 MHz.

FIG. 1

BLOOD

L.P.F

H.P.F

SUBTRACTER

LOGA-RITHMIC AMPLIFIER

LINEAR AMPLIFIER

DISPLAY

EP 0 417 796 B1

# FIG. 2

BLOOD

# FIG. 3

# FIG. 4